# EUROPEAN PATENT SPECIFICATION

(11) **EP 4 166 099 B1**
(45) Date of publication and mention of the grant of the patent: **11.02.2026**
(21) Application number: 22198248.1
(22) Date of filing: 28.09.2022
(51) Int. Cl.: A61B 17/122, A61B 17/24

(54) **EPISTAXIS CLIP**
EPISTAXIS-CLIP
PINCE D'ÉPISTAXIS

(30) Priority: 15.10.2021 US 202117502042
(43) Date of publication of application: 19.04.2023
(73) Proprietor: Freudenberg Medical, LLC, Carpinteria, CA 93013 (US)
(72) Inventor: Starkweather, Jennifer, Fort Myers, 33919 (US); Subramanya, Tejasvi, Oxnard, 93035 (US)
(74) Representative: Schaumburg und Partner Patentanwälte mbB

(56) References cited:
- CN-A- 105 476 685
- GB-A- 2 576 007
- NZ-A- 585 887
- US-A1- 2004 010 283
- US-A1- 2009 299 405
- US-A1- 2016 296 378
- US-B1- 6 666 211

## Description

### FIELD

The present disclosure relates to an epistaxis clip.

### BACKGROUND

This section provides background information related to the present disclosure which is not necessarily prior art.

Anterior epistaxis originates at the Kiesselbach's Plexus and accounts for between 90 and 95% of all nosebleeds. Anterior epistaxis is treated with compression and absorption. Many medical providers incorrectly treat anterior epistaxis, resulting in unnecessary and prolonged treatment and increased healthcare utilization. In particular, current treatment options and devices result in increased pain upon removal and re-aggravation of the wound and decreased cosmetic appeal. Additionally, some of the current treatment options also require pre-treatment or soaking, and completely occlude the nasal passages resulting in decreased or elimination of nasal breathing. Accordingly, it is desirable to provide an improved device for treating anterior epistaxis.

Document CN 105 476 685 A discloses a self-help device for stopping nasal bleeding. The device comprises a connecting piece, nose wing pads and a latch, wherein the nose wing pads are detachably mounted on the connecting piece; the latch is fixed on the connecting piece; the latch has opening and closed states; when the latch is in the closed state, the nose wing pads are in a bleeding stopping state. The self-help device for stopping nasal bleeding has multiple advantages that the latch is closed to drive the nose wing pads to press nose wings of a patient to stop bleeding, the patient is not required to press the nose wings by hand all the time, so that hands of the patient are liberated, and movement of the patient is facilitated; besides, the nasal cavity cannot be injured even by accidental touch because the nose wing pads are pressed on the outer side of the nose; the nose wing pads are mounted on the connecting piece detachably; different types and sizes of nose wing pads made of different materials can be changed conveniently; the nose wing pads can be adapted to different people through adjustment of the clamping tightness.

Document NZ 585 887 A discloses a device for absorbing excess nasal fluid is disclosed. The device, made from a unitary piece of absorbent material, comprises a body, a stop, and a pair of absorbent pads. The body comprises a pair of prongs where each prong is for positioning in a users nasal passage and the stop prevents further insertion of the prongs into the nasal passages. One of the pair of absorbent pads is positioned on each prong and configured to exert a gentle pressure upon the Kiesselbach's area of the nasal mucosa and absorb excess nasal fluid from the nasal passage.

Document GB 2 576 007 A discloses a nose clamp comprising a temperature altering body and a resilient clamping body with a bridge to extend over a dorsum of the user's nose and a first jaw and a second jaw extending from the bridge along the sidewalls of the user's nose to compress the nasal sidewalls and urge the temperature altering body into contact with the nasal sidewalls.

Document US 2004/010283 A1 discloses an apparatus for treatment of a nosebleed, in its preferred form having a pair of laterally spaced elongated arms pivotably connected to be selectively moved toward and away from each other like scissors or the like. A compression spring engages the arms to urge them into a desired position with respect to each other. Removable absorbent sponges are disposed on the upper ends of the arms and are arranged to engage both the septum of a nostril where typical nose bleeds would occur and the non-bleeding septum. The arms are squeezed to separate them and to insert the sponges into the nostrils and released to apply pressure on the bleeding and non-bleeding septums. The sponge engaging the bleeding area may be saturated with a vasoconstrictive agent to control the bleeding.

### SUMMARY

An object of the invention is to provide an epistaxis clip which exerts a certain pressure on the Kiesselbach's plexus.

This object is solved by an epistaxis clip with the features of claim 1. Advantageous embodiments are given in the dependent claims. The epistaxis clip is designed to apply predetermined pressure on the Kiesselbach's plexus. The pair of absorbent pads can include an absorbent foam that resists sticking to the wound.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure. An embodiment of the present invention are shown in FIGS. 1-4.
FIG. 1 is a perspective view of a epistaxis clip according to the principles of the present disclosure;
FIG. 2 is a perspective view of the epistaxis clip with the absorbent pads removed;
FIG. 3 is a side plan view of the assembled epistaxis clip according to the principles of the present disclosure;
FIG. 4 shows the epistaxis clip applied to a patient;
FIG. 5 is a perspective view of a epistaxis clip according to a second embodiment of the present disclosure; and
FIG. 6 is a perspective view of the epistaxis clip of FIG. 5 with the absorbent pads removed.

Corresponding reference numerals indicate corresponding parts throughout the several views of the drawings.

### DETAILED DESCRIPTION

Example embodiments will now be described more fully with reference to the accompanying drawings.

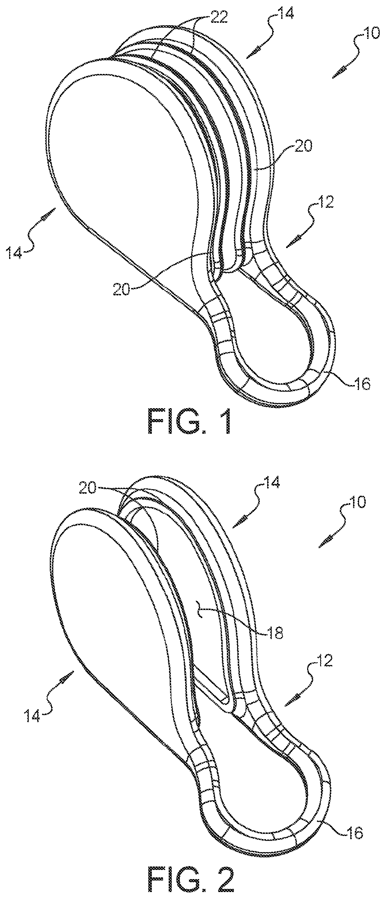

With reference to FIGS. 1-3 an epistaxis clip 10 is shown for applying predetermined pressure on the Kiesselbach's plexus in order to treat a nosebleed. The epistaxis clip 10 includes a frame structure 12 made from plastic or other engineering material. The frame structure 12 includes a pair of wings 14 that are connected to one another by a bridge portion 16 in a U-shaped configuration. Although the wings 14 are shown as being oblong or paddle shaped, other shapes can be used. The pair of wings 14 each define a pair of opposing recesses 18 surrounded by a rib 20 (best shown in Fig. 2). A pair of absorbent pads 22 are disposed in the respective opposing recesses 18 of the pair of wings 14. The absorbent pads 22 can be adhered or press-fit in the recesses 18. As the absorbent pads 22 absorb blood, they are allowed to expand further into the recesses 18. The absorbent pads 22 can also be replaceable within the frame structure 12 through either adhesive or press-fit.

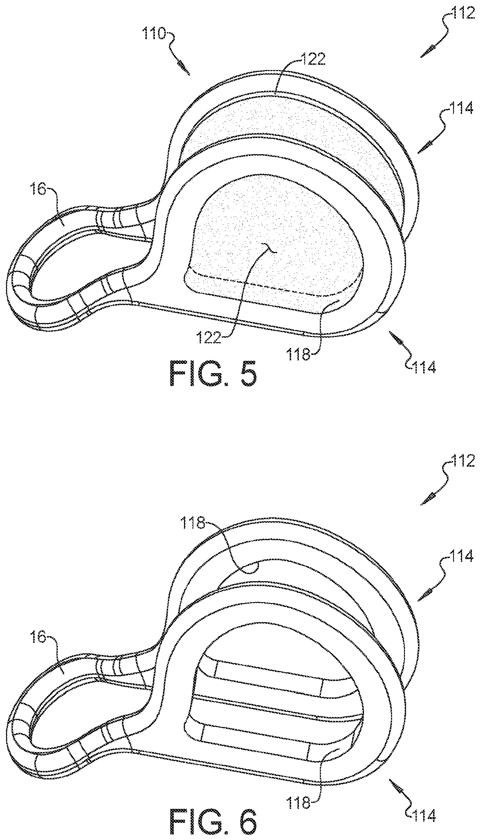

As an alternative to the recesses, the epistaxis clip 110 as shown in FIGS. 5 and 6 can include a frame structure 112 that can include a pair of through holes 118 through the wings 114 that receive the absorbent pads 122. In this embodiment, the absorbent pads can expand out through the through holes 118 as they absorb blood. FIG. 6 shows the frame structure 112 with the absorbent pads 122 removed for illustration purposes.

The absorbent pads 22,122 absorb blood at the site of the bleeding and can include a flexible silicone coated foam, a non-woven foam or a PVA that conform to the wound bed contouring and does not stick to the wound. The absorbent pads 22 can also be impregnated with Sodium Polyacrylate or other material for maximum absorption. In addition, the absorbent pads 22 can be provided with antimicrobial agents to avoid toxic shock syndrome and infection.

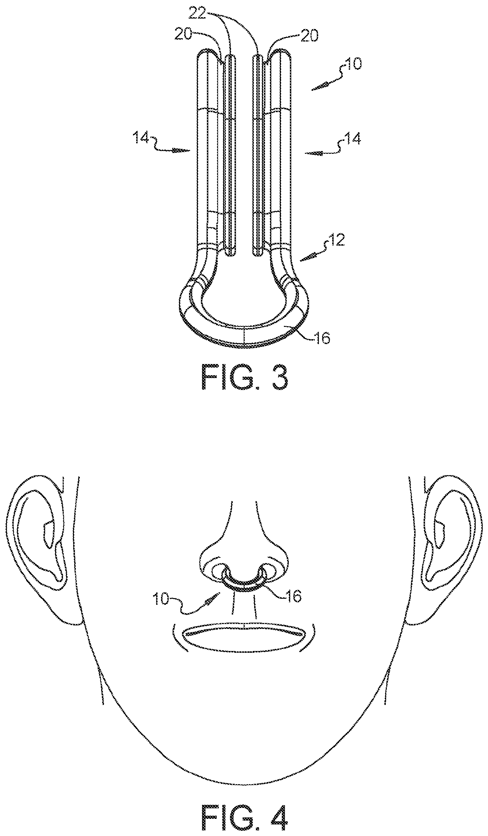

FIG. 4 shows the epistaxis clip 10 applied to the nostrils of a patient wherein the pair of wings 14 are applied to the patient's septum. The angle of the wings 14 applies compression at the Kiesselbach's plexus of the septum of at least 20 mmHg. When the bleeding has ceased, the epistaxis clip 10 can be removed from the nasal passages without fear of adhering to the wound or mucosa and discarded.

The pair of wings 14 of the epistaxis clip 10 are designed with a low profile so as not to occlude the nasal passages. The bridge portion 16 is formed from a clear plastic and extends from the nostrils below the columella. Because of the clear plastic, the bridge portion 16 is capable of blending in with the user's appearance so that it is more aesthetically appealing.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. An epistaxis clip (10), comprising:
a pair of wings (14) connected to one another by a bridge portion (16), the pair of wings (14) defining a pair of opposing recesses (18); and
a pair of absorbent pads (22) disposed in the respective opposing recesses (18) of the pair of wings (14), **characterized in that** the opposing recesses (18) are each defined by a raised rib (20).

2. The epistaxis clip according to claim 1, wherein the pair of absorbent pads (22) includes an absorbent foam.

3. The epistaxis clip according to claim 2, wherein the absorbent foam is impregnated with an absorbent material.

4. The epistaxis clip according to claim 1, wherein the wings (14) are oblong shaped.

5. The epistaxis clip according to claim 1, wherein the absorbent pads (22) are adhered in the recesses (18).

6. The epistaxis clip according to claim 1, wherein the absorbent pads (22) are press-fit in the recesses (18).

## Patentansprüche

1. Epistaxis-Klemme (10), umfassend:
ein Paar Flügel (14), die durch einen Brückenabschnitt (16) miteinander verbunden sind, wobei das Paar Flügel (14) ein Paar gegenüberliegender Aussparungen (18) definiert, und
ein Paar absorbierender Kissen (22), die in den jeweiligen gegenüberliegenden Aussparungen (18) des Paars Flügel (14) angeordnet sind, **dadurch gekennzeichnet, dass** die gegenüberliegenden Aussparungen (18) jeweils durch eine erhabene Rippe (20) definiert sind.

2. Epistaxis-Klemme nach Anspruch 1, wobei das Paar absorbierender Kissen (22) einen absorbierenden Schaumstoff aufweisen.

3. Epistaxis-Klemme nach Anspruch 2, wobei der absorbierende Schaumstoff mit einem absorbierenden Material imprägniert ist.

4. Epistaxis-Klemme nach Anspruch 1, wobei die Flügel (14) länglich geformt sind.

5. Epistaxis-Klemme nach Anspruch 1, wobei die absorbierenden Kissen (22) in den Aussparungen (18) verklebt sind.

6. Epistaxis-Klemme nach Anspruch 1, wobei die absorbierenden Kissen (22) in die Aussparungen (18) eingepresst sind.

## Revendications

1. Pince d'épistaxis (10), comprenant :
une paire d'ailes (14) reliées l'une à l'autre par une partie de pont (16), la paire d'ailes (14) définissant une paire d'évidements opposés (18) ; et
une paire de tampons absorbants (22) disposés dans les évidements opposés respectifs (18) de la paire d'ailes (14), **caractérisée en ce que** les évidements opposés (18) sont définis chacun par une nervure en relief (20).

2. Pince d'épistaxis selon la revendication 1, dans laquelle la paire de tampons absorbants (22) inclut une mousse absorbante.

3. Pince d'épistaxis selon la revendication 2, dans laquelle le tampon absorbant est imprégné d'un matériau absorbant.

4. Pince d'épistaxis selon la revendication 1, dans laquelle les ailes (14) sont de forme oblongue.

5. Pince d'épistaxis selon la revendication 1, dans laquelle les tampons absorbants (22) sont mis en adhérence dans les évidements (18).

6. Pince d'épistaxis selon la revendication 1, dans laquelle les tampons absorbants (22) sont ajustés par pression dans les évidements (18).
